# EUROPEAN PATENT SPECIFICATION

(11) **EP 2 488 116 B1**
(45) Date of publication and mention of the grant of the patent: **22.11.2017**
(21) Application number: 10760695.6
(22) Date of filing: 04.10.2010
(51) Int. Cl.: A61B 17/34, A61M 27/00, A61M 25/06, A61M 39/22, A61M 39/06, A61M 39/10

(54) **MEDICAL DEVICE FOR APPLYING CATHETERS, PARTICULARLY FOR THORACENTESIS PROCEDURES**
MEDIZINISCHE VORRICHTUNG ZUR ANWENDUNG VON KATHETERN, IM BESONDEREN FÜR PLEURAPUNKTIONSVERFAHREN
DISPOSITIF MÉDICAL POUR L'APPLICATION DE CATHÉTERS, EN PARTICULIER POUR DES PROCÉDURES DE THORACENTÈSE

(30) Priority: 15.10.2009 IT MI20091772
(43) Date of publication of application: 22.08.2012
(73) Proprietor: Gibertoni, Lucio, 41037 Mirandola (Modena) (IT); Gibertoni, Andrea, 41039 San Possidonio (IT)
(72) Inventor: Gibertoni, Lucio, 41037 Mirandola (Modena) (IT); Gibertoni, Andrea, 41039 San Possidonio (IT)
(74) Representative: Modiano, Micaela Nadia
(86) International application number: PCT/EP2010/064761
(87) International publication number: WO 2011/045196

(56) References cited:
- WO-A1-01/78809
- WO-A1-2007/073939
- DE-U1-202007 000 173
- GB-A- 2 185 689
- US-A- 3 157 201
- US-A- 3 572 375
- US-A- 4 013 080
- US-A- 4 588 402
- US-A- 4 610 671
- US-A- 5 300 046
- US-A- 5 396 899
- US-A- 5 409 462
- US-A- 6 146 374
- US-A1- 2007 066 965

## Description

### Technical Field

The present invention relates to a medical device for applying catheters, particularly for thoracentesis procedures.

### Background art

Thoracentesis is a procedure adapted to remove fluids, both in the liquid state and in the gaseous state, from the pleural space by means of a large needle or a small cannula made of plastics, termed more commonly catheter, which can be inserted in the needle or more commonly covers such needle so as to leave it connected to the pleural space once the needle has been removed.

More precisely, the procedure entails retracting the metal tip of the needle from the catheter as soon as the tip of the catheter makes contact with the liquid to be drained. Subsequently, one advances a little further inside the pleural space and the entire metallic portion is retracted from the catheter, which is pushed completely inside the pleural space so as to allow the complete evacuation of the liquid without the risk of piercing the lung.

The catheter is inserted in the body of the patient by performing a small cut, measuring by way of indication 2 millimeters, in the skin of the patient with the tip of a scalpel in order to prevent the catheter itself, which covers the metallic needle, from gathering on the needle while it attempts to penetrate the skin.

In latest-generation medical devices of the known type adapted to manage and apply catheters, inside the sharp-tipped needle, accommodated in the cannula made of plastics, there is a round-tipped element, which is spring-loaded and protrudes beyond the sharp tip of the needle and retracts into the needle while the thoracic wall is pierced. As soon as the wall is passed, i.e., as soon as there is no more resistance, by action of the spring the round-tipped element protrudes again past the sharp tip of the needle.

This solution is advantageous because as the liquid is evacuated, the lung, by expanding, comes into contact with the tip of the round-tipped element and not with the tip of the sharp-tipped needle, thus avoiding accidental puncture of said lung.

Generally, the system just described is supported by a hollow supporting element with three openings, two of which are mutually opposite, to which the plastic cannula, a syringe for drugs and a bag for collecting the liquid are connected respectively.

More precisely, inside the supporting element there is a manually actuated selector by means of which it is possible to connect the plastic cannula, associated with the first opening of the supporting element, to one or both of the remaining openings.

Practically, when the catheter is inserted in the pleural space, the opening that lies opposite the one associated with the catheter is free so as to allow the extraction of the sharp-tipped needle once the thoracic wall of the patient has been pierced and the catheter has been oriented correctly inside the pleural space.

In fact, during the step of applying the catheter, it is possible to orient the latter inside the pleural space by directly moving the sharp-tipped needle that is present therein.

After the extraction of the sharp-tipped needle, it is possible to connect the syringe to the supporting element, if necessary.

Differently, the collection bag is connected immediately to the supporting element, at the opening that is not opposite the one associated with the catheter, so as to collect immediately the liquid to be drained that exits from the patient also during the steps for applying the catheter.

These know types of medical device are not devoid of drawbacks, which include the fact that once the catheter has been applied to the patient, when the catheter is no longer able to aspirate the liquid to be drained because of the lowering of the latter in the pleural space, the catheter must be removed and replaced with another one, inserted with a new orientation, so as to draw the remaining portion of the liquid to be drained.

Another drawback consists in that during the process for aspirating the fluid, by means of a syringe, currently commercially available products force the operator to switch the flow diverter valve at every single stroke of the piston of the syringe. In other words, during suction (traction of the piston in the extended position) the selector must be in a specific position for connecting the syringe proper to the thorax of the patient; during the return step of the piston to the initial position, the selector must be switched to a different position so as to connect the syringe to the collection bag.

Selector switching must be performed with each suction and discharge, until the end of the drainage operation. Another drawback of existing products is the presence of the one-way valve in the grip of the sharp-tipped needle. This is to allow unidirectional suction of a small quantity of liquid and to verify the positioning of the needle inside the bag of fluid to be drained, before extracting such bag.

This leads to an increase in the dimensions of such grip, with greater handling difficulty. Moreover, exploratory suction is performed with a syringe different from the one that will be used subsequently for the drainage, i.e., a smaller one, with the consequent need to supply it with the product and an increase in the degree of complexity of the kit and its cost.

Another drawback of medical devices of the known type consists in that the medical staff assigned to apply the catheter does not have any feedback on the position of the catheter inside the body when it is applied, i.e., whether the catheter has already entered the pleural space or is still passing through the tissues of the ribcage.

In other words, the medical staff assigned to apply the catheter, with medical devices of the known type, does not have any feedback on the position of the round-tipped element with respect to the sharp-tipped needle, i.e. on whether the former is protruding from the latter or is retracted completely or partially inside the latter.

DE 202 007 000 U1 discloses a drainage catheter of the conventional type as described above, with a needle cannula slidingly arranged through a three-way valve and in the catheter through an opening of a resilient sealing material plug. The opening is formed when the needle is inserted through the plug and through the catheter, and when the needle is removed the plug automatically closes so that no liquid may escape.

US 5 300 046 A also discloses a conventional thoracentesis sheath catheter assembly of the type a described above, having a catheter connected to a valve body provided with a throughbore coaxial to the catheter for receiving a thoracentesis needle therethrough and a drainage bore in fluid communication with the first thoughbore. A ball valve automatically establishes a fluid path to the drainage bore when the needle is removed, a valve with one or more luer adapters being connected to the drainage bore for connection to one of more drainage bags.

US 3 572 375 A discloses a twin valve T-connector provided with three central passages two of which are provided with one-way check valves for fluid flow in one direction and the other of which has a syringe connected thereto, for selectively injecting fluids into or withdrawing fluids from a body.

### Disclosure of the invention

The aim of the present invention is to provide a medical device for applying catheters, particularly for thoracentesis procedures, which allows repositioning of the catheter inside the pleural space after the application steps without necessarily extracting and reapplying the catheter as in the background art,

Within this aim, an object of the present invention is to provide a medical device for applying catheters that gives constant feedback on the position of the catheter inside the body during its application.

Another object of the present invention is to provide a medical device for applying catheters that is structurally simple and whose manufacturing costs are competitive with respect to those of the background art.

In accordance with the invention, there is provided a medical device for applying catheters, particularly for thoracentesis procedures, as defined in the appended claims.

### Brief description of the drawings

Further characteristics and advantages of the present invention will become better apparent from the description of two preferred but not exclusive embodiments of a medical device for applying catheters, according to the invention, illustrated by way of non-limiting example in the accompanying drawings, wherein:

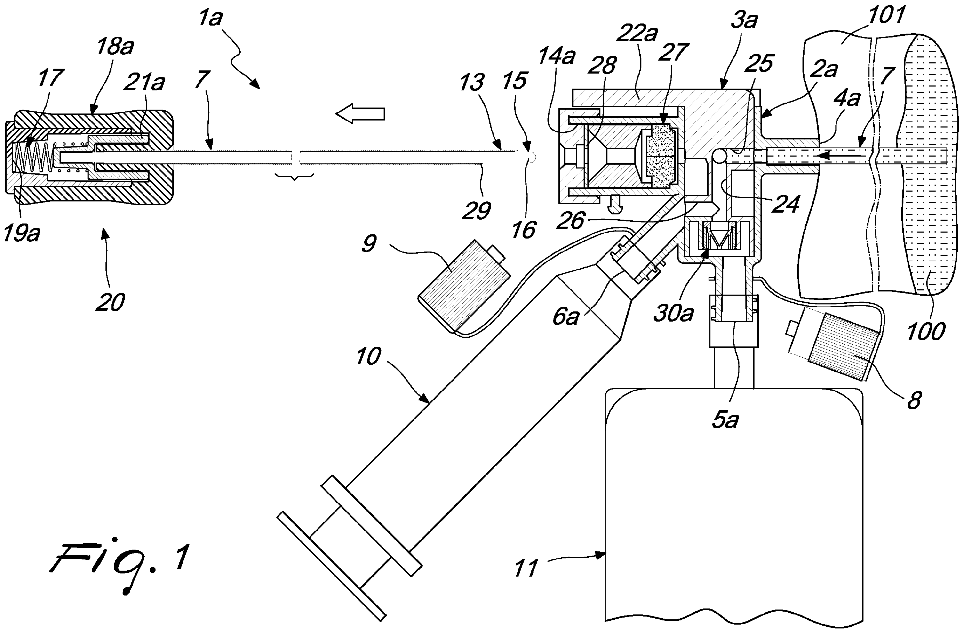

Figure 1 is a partially sectional side elevation view of the first embodiment of the medical device for applying catheters, particularly for thoracentesis procedures, according to the invention;

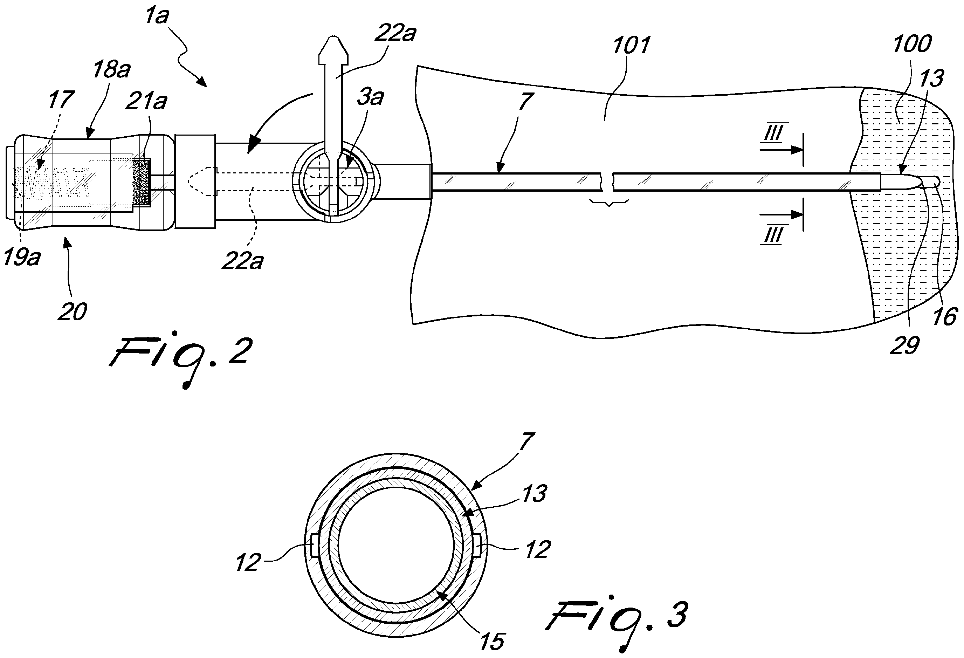

Figure 2 is a plan view of the medical device for applying catheters according to the invention;
Figure 3 is a sectional view, taken along the line III-III, of the medical device illustrated in Figure 2;

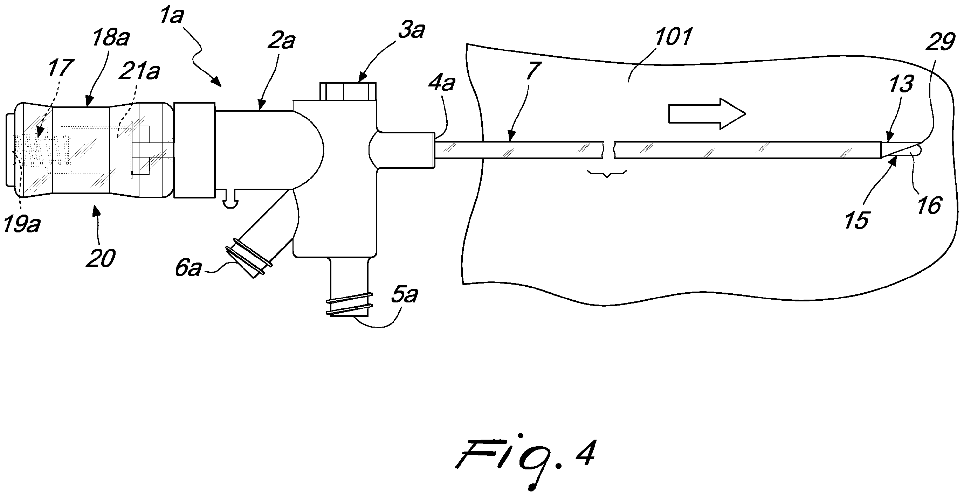

Figure 4 is a partially sectional side elevation view of the medical device illustrated in Figure 1 during the step of insertion of the catheter through the tissues that lie in the vicinity of the biological liquid to be drained;

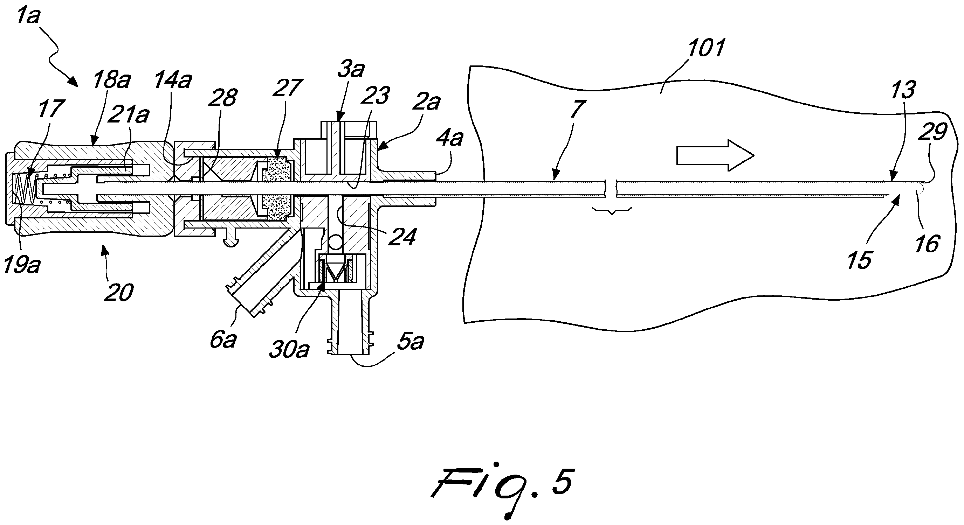

Figure 5 is a longitudinal sectional view of the medical device illustrated in Figure 4;

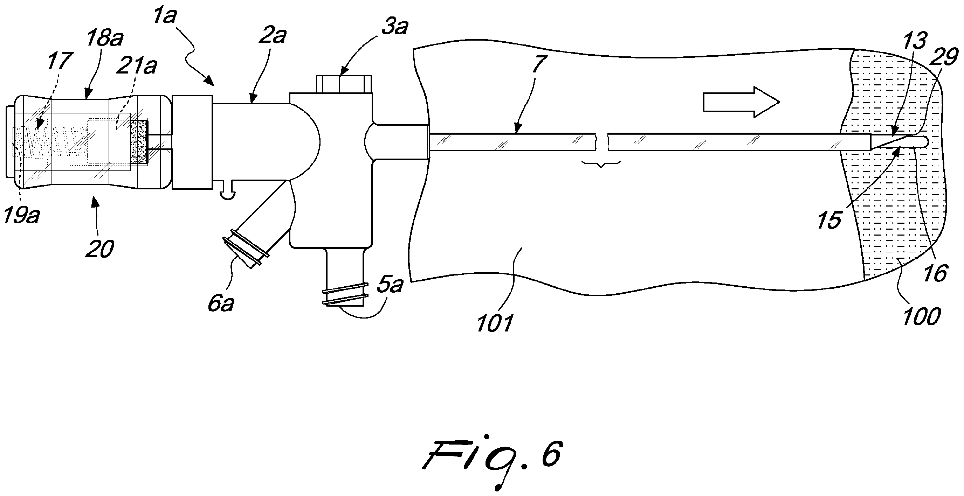

Figure 6 is a partially sectional side elevation view of the medical device illustrated in Figure 1 at the end of step of inserting the catheter;

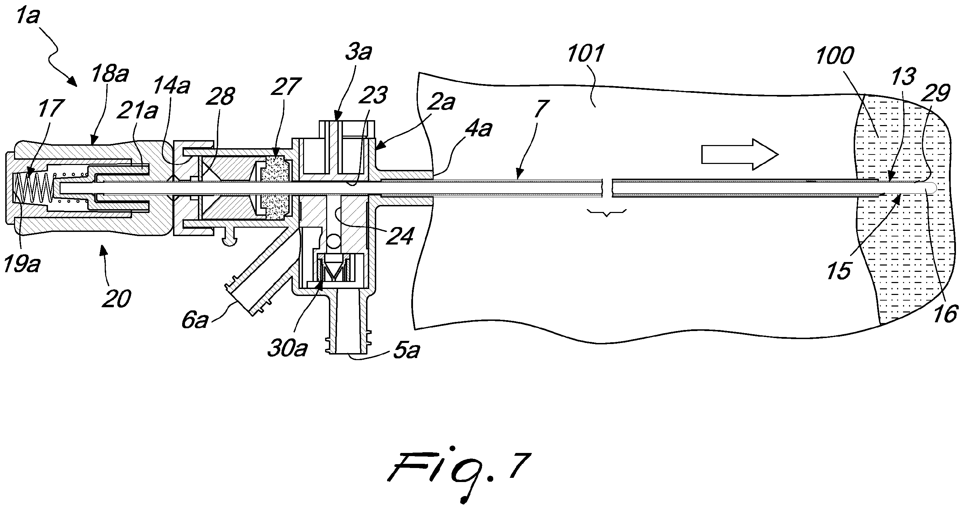

Figure 7 is a longitudinal sectional view of the medical device illustrated in Figure 6;
Figures 8 and 9 are two partially sectional side elevation views of the second embodiment of the medical device for applying catheters, particularly for thoracentesis procedures, according to the invention;
Figure 10 is a perspective view of the medical device illustrated in Figures 8 and 9 during the step of insertion of the catheter through the tissues that lie in the vicinity of the biological liquid to be drained;
Figure 11 is a perspective view of the medical device illustrated in Figures 8 and 9 during the step of draining the biological liquid to be drained;
Figures 12 and 13 are two perspective views of the medical device illustrated in Figures 8 and 9 during the step of repositioning the catheter;
Figures 14 and 15 are two perspective views of the medical device illustrated in Figures 8 and 9 during the step of draining the biological liquid to be drained.

### Ways of carrying out the invention

With reference to the figures, the medical device for applying catheters, particularly for thoracentesis procedures, is generally designated in the two proposed embodiments by the reference numerals 1a and 1b.

Hereinafter, the elements that are common to both embodiments will be designated by the same numbering and the elements that are present in both embodiments but have geometric variations between one embodiment and the other will be numbered by adding the letter "a" as suffix for the first embodiment and the letter "b" as suffix for the second embodiment.

The medical device 1a or 1b comprises a hollow supporting element 2a or 2b, which can be gripped ergonomically and accommodates a selector 3a or 3b, which is operated manually and is adapted to connect at least three openings 4a, 5a and 6a or 4b, 5b and 6b defined in the supporting element 2a or 2b.

More precisely, in both of the proposed embodiments, the first opening 4a or 4b is functionally associated with a cannula 7, which is adapted to be inserted in the body of a patient to drain biological liquid 100 or to inject drugs, and the second opening 5a or 5b and the third opening 6a or 6b, which have standard connectors for example of the female LUER-LOCK type, can instead be functionally associated respectively with two closure elements 8 and 9 or with at least one of a syringe 10 and a collection bag 11, which can have a reservoir made of silicone for the forced aspiration of the biological liquid 100 to be drained.

Advantageously, it is possible to insert slidingly in the cannula 7, which can comprise at least one groove 12 that is provided on its inner surface and runs longitudinally with respect to the cannula 7 for the constant connection of the first opening 4a or 4b to the biological liquid 100 to be drained, a sharp-tipped needle 13 whose length is such as to pass through at least the entire cannula 7 and protrude from it so as to pierce the biological tissue 101 that lies in the vicinity of the biological liquid 100 to be drained.

According to the invention, each one of the supporting elements 2a and 2b forms, on the side opposite the first opening 4a or 4b, a fourth opening 14a or 14b for extraction or insertion of the sharp-tipped needle 13, respectively, from and into the cannula 7 without involving the openings 5a or 5b and 6a or 6b.

In other words, in this manner an opening is provided which is dedicated only to the operations of extraction and insertion of the sharp-tipped needle 13, which can be moved without necessarily detaching any syringes 10 and collection bags 11 connected to the supporting element 2a or 2b.

More precisely, a round-tipped element 15 is inserted slidingly in the sharp-tipped needle 13 so that its round tip 16 normally protrudes from the sharp-tipped needle 13 on the side opposite the supporting element 2a and 2b. Such round tip 16 is retractable into the sharp-tipped needle 13 in opposition to the action of elastic means 17 associated with the round-tipped element 15 and following the impact of the round tip 16 against a body that has such a consistency as to make it retract, such as, for example, the biological tissue 101 that lies in the vicinity of the pleural space.

As regards the elastic means 17, they comprise a helical spring, which is accommodated in a container body 18a or 18b, which is jointly connected to the sharp-tipped needle 13 and can engage the supporting element 2 at the fourth opening 14a or 14b.

More precisely, the helical spring of the elastic means 17 is interposed between one end of the round-tipped element 15 and an internal abutment surface 19a or 19b of the container body 18a or 18b.

Advantageously, for having a visual feedback of the retraction of the round tip 16 into the sharp-tipped needle 13 during use of the medical device 1, visualization means 20 are provided which comprise a slider 21a or 21b, associated with the round-tipped element 15 on the side opposite the round tip 16.

The slider 21a or 21b is accommodated in the container body 18a or 18b and can move from a visible position, in which the round tip 16 protrudes at least partially from the sharp-tipped needle 13, to a non-visible position, in which the round tip 16 is completely retracted into the sharp-tipped needle 13 as a consequence of the translation of the round tip 16.

Conveniently, this visibility is made possible by the fact that the container body 18a or 18b is at least partially or totally transparent for viewing the slider 21a or 21b in its visible position.

Moreover, the container body 18a or 18b has such a shape that it can be gripped easily by medical staff so as to facilitate the operations of insertion and extraction of the sharp-tipped needle 13 in and from the cannula 7.

More precisely, both the supporting element 2a and 2b and the container body 18a and 18b in the first embodiment and in the second embodiment have reduced dimensions for increasing the comfort of the patient during prolonged uses, i.e., in cases in which the medical device 1b has to remain in place for several days. Obviously, the reduced dimensions must not be detrimental to the grip, which must be ergonomic for providing optimum control during the positioning step.

In particular, the supporting element 2b, with respect to the supporting element 2a, eliminates all unnecessary spaces, making it even more compact, and this allows easy use to the physician and, at the same time, a practical fixing in case of long-term applications.

Moreover, the container body 18b has such a geometric shape as to facilitate its gripping by the operating physician. This shape has in fact two mutually opposite concavities, such as to accommodate part of the physician's fingers.

As will be described in more detail hereinafter, during use of the medical device 1a or 1b the four openings 4a, 5a, 6a and 14a or 4b, 5b, 6b and 14b can be mutually connected selectively by means of the selector 3a or 3b, which has manually actuated control means, consisting of a control lever 22a or 22b that can move from a first functional position to a second functional position.

More precisely, the four openings 4a, 5a, 6a and 14a or 4b, 5b, 6b and 14b are all four mutually connected when the control lever 22a or 22b is arranged in the first functional position.

When the control lever 22a or 22b is arranged in the second functional position, only the first opening 4a or 4b, the second opening 5a or 5b and the third opening 6a or 6b are instead mutually connected.

More particularly, each one of the selectors 3a and 3b consists of a small cylinder inserted in the respective supporting element 2a or 2b substantially at right angles to the insertion axis of the sharp-tipped needle 13 and can rotate about its own axis, thanks to the control lever 22a or 22b, so as to switch it from the first functional position to the second functional position and vice versa.

For further improving the ergonomics of the medical device 1b, the control lever 22b has a flap 50 that protrudes along a direction substantially parallel to the axis of the small cylinder of the selector 3b that is inserted in the supporting element 2b, so as to be gripped easily by the physician.

With reference to the first functional position, the selector 3a or 3b has a first duct 23 that passes entirely through it and connects the first opening 4a or 4b and the fourth opening 14a or 14b.

A second duct 24 branches off from the first duct 23, whose dimensions are such as to allow the passage of the sharp-tipped needle 13, at right angles to said first duct 23 in the direction of the second and third openings 5a or 5b and 6a or 6b to which it is connected.

More precisely, with the selector 3a or 3b arranged in the first functional condition, the openings 5a or 5b and 6a or 6b are connected to each other and the second duct 24 is connected only to the second opening 5a or 5b.

With reference to the second functional position, the selector 3a or 3b has a third duct 25, which is adapted to connect the first opening 4a or 4b to the second duct 24, which in turn is connected to the second opening 5a or 5b.

In this second functional position, the openings 5a or 5b and 6a or 6b are not connected directly to each other but are both connected to the third duct 25, respectively, through the second duct 24, which is connected directly to the second opening 5a or 5b and to the third opening 6a or 6b by means of a fourth duct 26, which is connected to the second duct 24.

In this manner, the fourth opening 14a or 14b is isolated from the other three openings 4a, 5a and 6a or 4b, 5b and 6b.

Advantageously, in both of the supporting elements 2a and 2b there is a first valve 27 of the one-way normally-closed type, which is interposed between the respective selector 3a or 3b and the respective fourth opening 14a or 14b and has a membrane-type sealing gasket 28 which can engage by abutment against the respective container body 18a or 18b if present.

More precisely, this first valve 27 has a central slit which is elastically deformable for the passage of the sharp-tipped needle 13 in both directions so as to make it possible to insert it and extract it from the cannula 7.

In order to avoid leaks due to the difference in diameter and shape between the sharp-tipped needle 13, its sharp part 29 and the round tip 16 of the round-tipped element 15, the first valve 27 and the membrane-type sealing gasket 28 are arranged at a distance from each other that is greater than the sum of the length of the sharp part 29 of the sharp-tipped needle 13 and the stroke of the round tip 16 of the round-tipped element 15.

With this solution, it is possible to prevent unwanted leaks of the biological liquid 100 to be drained during the operations for inserting and extracting the sharp-tipped needle 13 in and from the cannula 7.

In both of the supporting elements 2a and 2b, a respective second valve 30a or 30b of the one-way type is accommodated and interposed between the respective selector 3a or 3b and the second opening 5a or 5b.

More precisely, in the first embodiment and in the second embodiment the second valve 30a and 30b consists of an umbrella valve that has a reduced vertical size, promoting the compactness of the supporting element 2b.

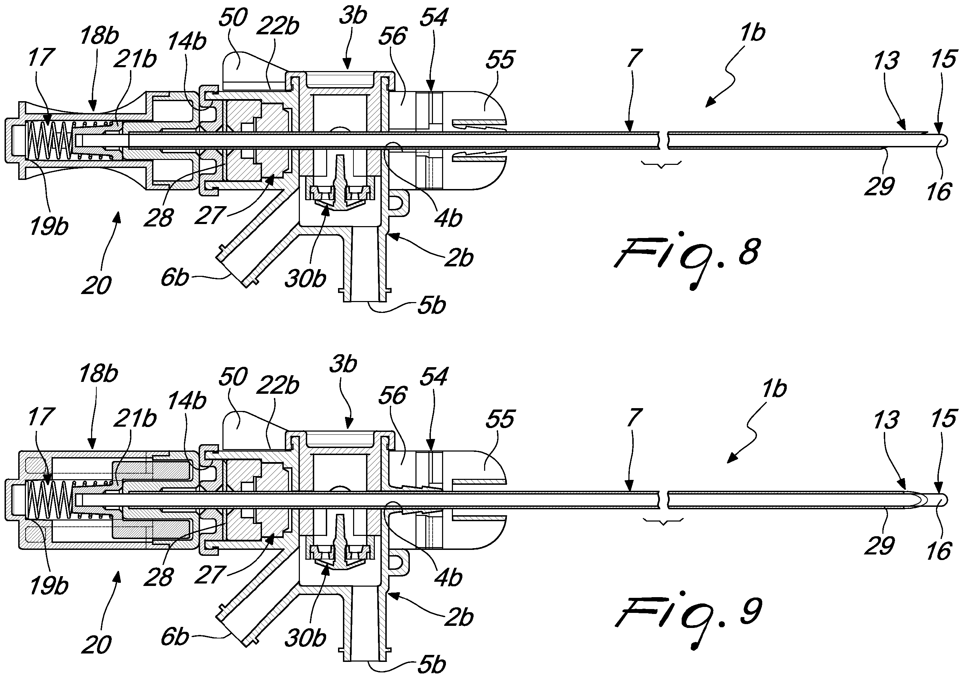

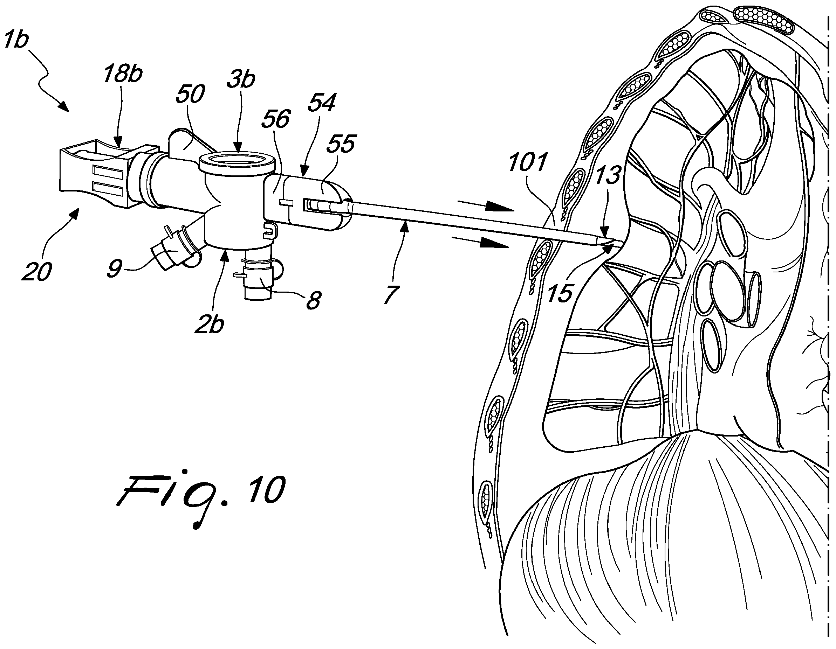

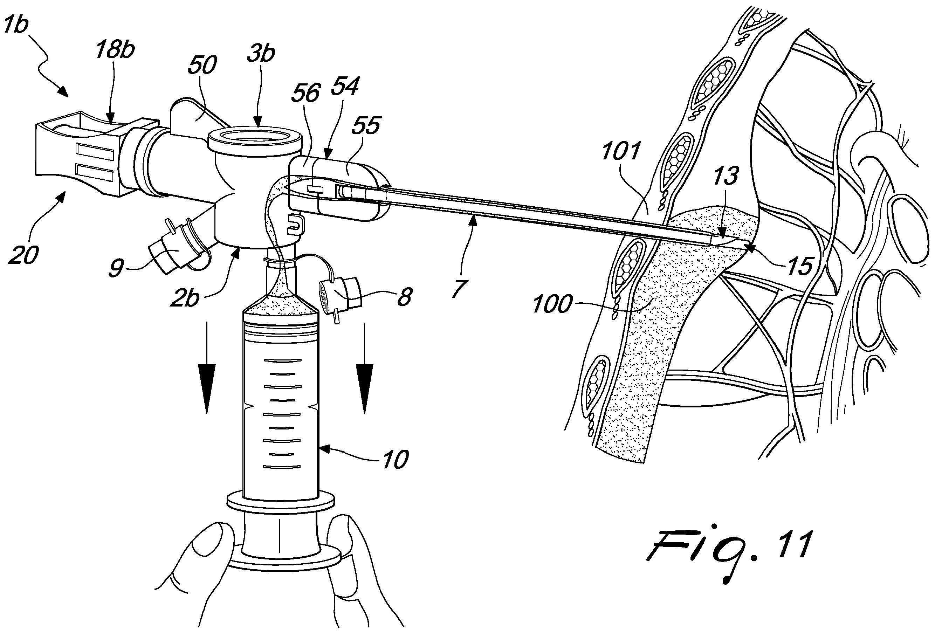

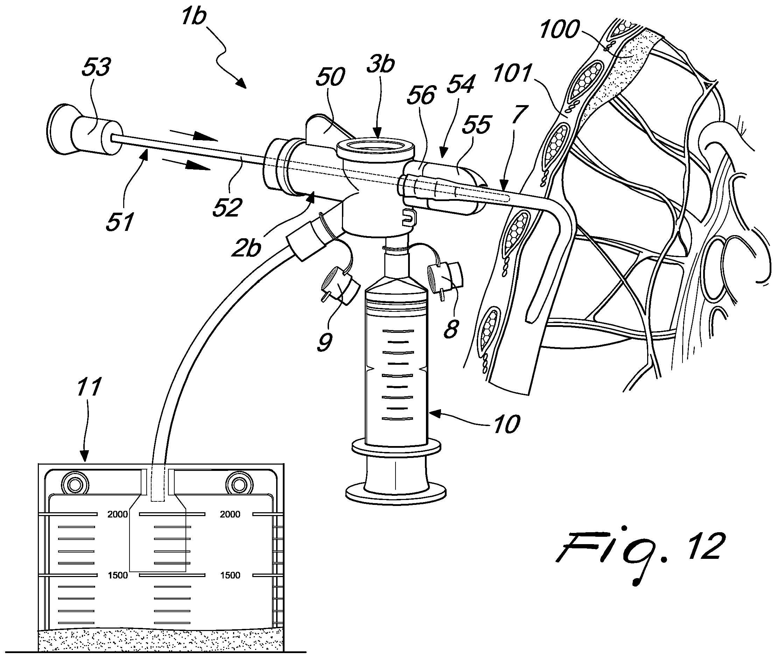

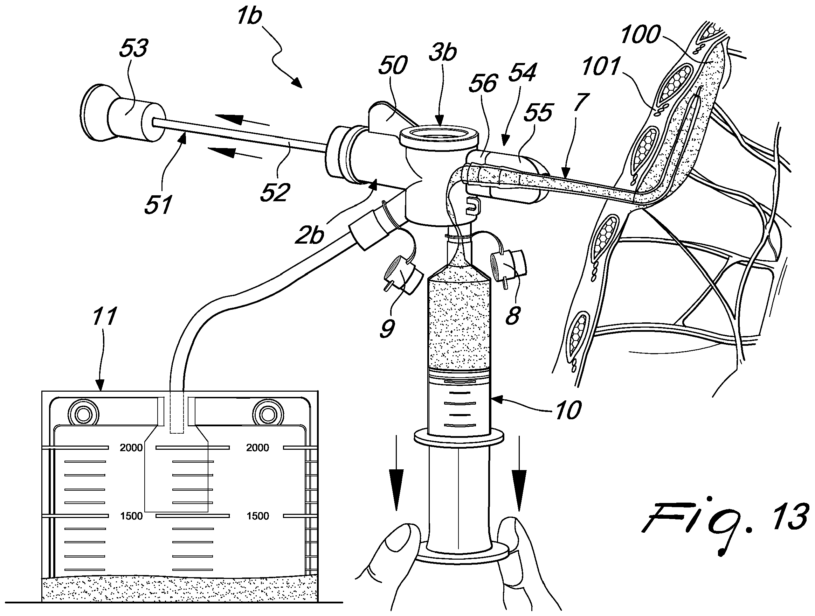

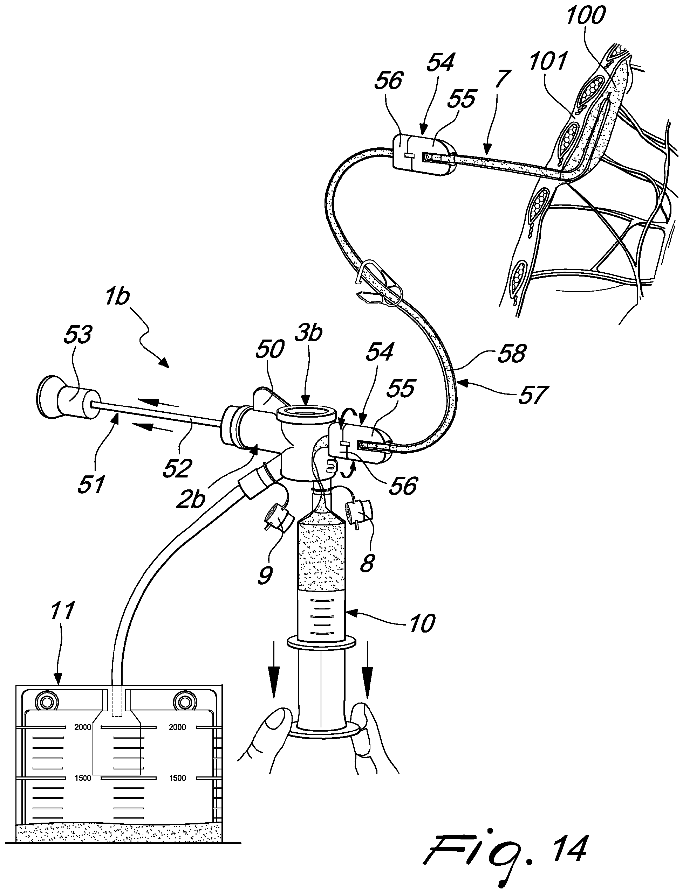

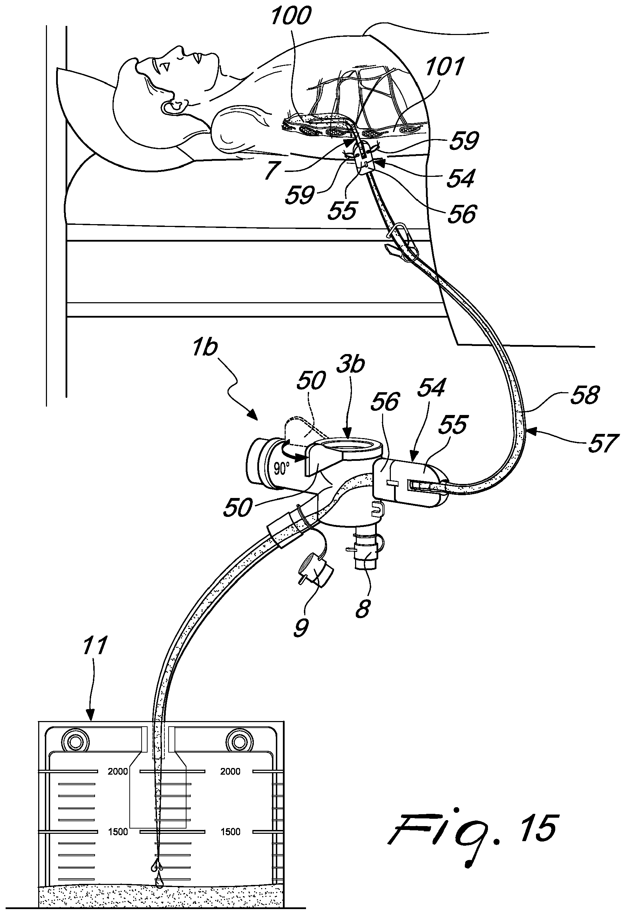

With reference to Figures 8 to 15, if there are pockets of fluid to be drained in different places in the patient, the cannula 7 would have to be inserted again with a different orientation from the previous one, so as to drain effectively all the fluid present in the pleural space or in other body cavities.

As will be described in more detail hereinafter, in order to allow the repositioning of the medical device 1b, as an alternative to the sharp-tipped needle 13 there is a spindle 51, which consists substantially of a small round-tipped rod 52 and has, at the end that lies opposite the round tip, a grip body 53.

The spindle 51 can be used also in the first embodiment so as to allow the repositioning of the medical device 1a.

In order to further improve the comfort of the patient if the medical device 1b must remain inserted in place for a prolonged time, it is possible to space the supporting element 2b of the medical device 1b from the exit point of the catheter, in such a position as to not cause discomfort to the patient or damage to the skin, by means of one or more connecting elements 54.

More precisely, the connecting elements 54 comprise a male element 55 and a female element 56, which can be mutually associated by means of a coupling of the bayonet type and can be fixed respectively to the cannula 7 and to the opening 4b of the supporting element 2b.

As will be described better hereinafter, extension elements 57 can be provided which are constituted by a small tube 58 that has, at its ends, the elements 55 and 56 so that it is interposed between the cannula 7 and the supporting element 2b.

For keeping the medical device 1b applied for a prolonged period of time, avoiding the accidental risk that the catheter might move from the original position or even be extracted eyelet elements 59 are provided on the supporting element 2b and on the connecting elements 54.

In this manner is possible to easily fix these parts of the medical device 1b to the skin of the patient, thus avoiding possible displacements of the position of the catheter or even accidental extraction thereof.

Application and operation of the medical devices 1a and 1b for applying catheters, particularly for thoracentesis procedures, are described hereinafter.

By gripping the medical device 1a or 1b by the supporting element 2a or 2b, the medical staff assigned to the application of the medical device 1a or 1b arranges said device so that the round tip 16 is directed toward the part of the thorax on which insertion is to be performed.

This insertion, which occurs generally on the rear part of the thorax just below the tip of the scapula, approximately in the eighth intercostal space, occurs by pushing forcefully the entire medical device 1a or 1b against the thorax of the patient, for example, by pushing the container body 18a or 18b.

This pressure causes the round tip 16 to retract into the sharp-tipped needle 13, leaving the sharp part 29 of the sharp-tipped needle 13 free to pierce the skin and the muscular tissues of the patient until the pleural space of the patient is reached.

This arrival can be ascertained by checking whether the slider 21a or 21b is visible in the container body 18a or 18b.

In the medical device the slider is in fact clearly visible before use. When instead the sharp-tipped needle 13 penetrates "hard" tissues, the slider 21a or 21b disappears behind the container body 18a or 18b. As soon as the sharp part 29 of the sharp-tipped needle 13 enters the pleural space, the round tip 16, since it no longer has a force which contrasts and opposes the force applied by the elastic means 17, exits again and with it the slider 21a or 21b returns to its visible position, so as to allow the medical staff to understand that piercing has been completed.

With such a solution, i.e., with the round tip 16 that exits again from the sharp-tipped needle 13 as soon as a "soft" part is reached, one avoids damaging tissues such as, for example, pulmonary tissue with the sharp portion 29 of the sharp-tipped needle 13.

Once the sharp-tipped needle 13, which carries the cannula 7 with it as well, has been inserted, it is possible to connect a syringe 10 and a collection bag 11 to the respective openings 5a and 6a or 5b and 6b.

This connection can also be performed before piercing of the patient's thorax.

In any case, by means of the groove or grooves 12 it is possible to check whether the tip of the cannula 7 leads into the pleural space as desired.

In fact, the biological liquid 100 to be drained reaches, by way of the grooves 12, the first opening 4a or 4b and from there, by way of the ducts formed by the selector 3a or 3b arranged in the first functional position, it arrives, through the one-way valve 30a or 30b, at the opening 5a or 5b to which a syringe is connected by means of which it is possible to check the flow of the drained biological liquid 100.

If the biological liquid 100 does not flow as described, the medical device 1a or 1b is oriented until the cannula 7 draws the biological liquid 100 correctly.

Once it has been ascertained that the piercing operation has been performed correctly, the sharp-tipped needle 13 is extracted from the medical device 1 by removing it and all the components connected thereto.

During this removal, there are no unwanted leaks because, as already mentioned, the first valve 27 and the membrane-type sealing gasket 28 are arranged at a distance from each other that is greater than the sum of the length of the sharp portion 29 of the sharp-tipped needle 13 and the stroke of the round tip 16 of the round-tipped element 15.

Once the sharp-tipped needle 13 has been extracted, leaving the selector 3a or 3b in its first functional position, the cannula 7 is connected to the inside of the selector 3a or 3b, but the presence of the one-way valve 30a or 30b and the valve 27 makes it possible to prevent the flow of air from the environment back toward the pleural space of the patient, allowing staff to operate in complete safety.

In order to drain the biological liquid 100, it is possible to connect the syringe to the opening 5a or 5b and a collection bag to the opening 6a or 6b. When the piston of the syringe is retracted, the one-way valve 30a or 30b allows the liquid to pass and be collected in the syringe proper. When the piston of the syringe is pushed into its initial position, the one-way valve 30a or 30b prevents the biological liquid from flowing back toward the patient and thus such liquid is conveyed to the opening 6a or 6b and collected in the bag. The aspiration and collection operation, by acting on the piston of the syringe 10, can be performed several times without the need to act on the position of the selector, thus simplifying the execution of the procedure.

By rotating the selector 3a or 3b to its second functional position, the opening 14a or 14b is isolated, leaving in direct connection the other openings 4a and 6a or 4b and 6b while the opening 5a or 5b is isolated by the one-way valve 30a or 30b. In this manner, the biological liquid 100 can be collected directly by passing through the cannula 7, through the ducts 25, 24 and 26 toward the opening 6a or 6b, avoiding passage through the one-way valve 30a and 30b. In this manner, load losses caused by the one-way valve 30a or 30b are reduced, facilitating the outflow and collection of the biological liquid 100 by gravity.

If the level of the biological liquid 100 to be drained inside the pleural space drops below the drawing end of the cannula 7, it is possible by repositioning the control lever 22a or 22b in its first functional position to reinsert the sharp-tipped needle 13 in the cannula 7 so as to reorient it in the pleural space, so as to draw the remaining portion of the biological liquid 100 to be drained and repeat the procedure just described without however disconnecting the cannula 7 from the patient's body.

As an alternative, for positioning of the medical device 1a or 1b, the spindle 51 can, at any time, be inserted from the opening 14a or 14b, performing simultaneously the partial extraction of the catheter.

With the spindle 51 inserted, but without complete extraction of the catheter, such catheter can be oriented in a different position, affecting the pocket where the fluids to be drained are located.

At this point, the catheter can be inserted completely and the spindle 51 can be extracted from the supporting element 2a or 2b.

The operation is entirely similar to the one performed during initial positioning, and therefore the physician does not have to learn different maneuvers, to the full benefit of ease of use of the medical device 1a or 1b.

Moreover, the use of a spindle 51 makes the procedure completely safe, avoiding any damage to pulmonary tissue.

Finally, the impossibility of connecting the thorax to the outside environment no matter what maneuver the physician performs makes it possible to change the position of the catheter whenever necessary, without limitations of a technical type to the number of repositionings.

Often catheter access, in thoracentesis procedures, is performed from the back, and therefore when the catheter is inserted completely the body is in such a position as to make it very difficult for the patient to stay in the supine position.

Thanks to the presence of extension elements 57 it is possible to move the fixing of the supporting element 2b with respect to the exit point of the catheter.

If it is necessary to fix the supporting element 2b of the medical device 1b in another position than the exit point of the catheter, it is possible to disconnect the cannula 7 and the supporting element 2b in order to insert one or more extension elements 57, mutually spacing the parts.

The bayonet couplings of the elements 55 and 56, of the male and female type, make it possible to perform the operation simply and rapidly and leave unchanged the properties of the medical device 1b of being connectable to different gravity- or suction-based collection systems.

Once the unit has been assembled, the end that lies proximate to the end portion of the cannula 7, i.e., where such cannula exits from the body of the patient, can be fixed to the skin by means of ordinary suture stitches.

The other end of the extension line, i.e., the one connected to the supporting element 2b, can be in turn fixed in such a position as to allow the patient to assume different positions comfortably without feeling pain or discomfort and at the same time allow access and connection of the collection devices by medical staff.

The importance of the insertion of the extension elements 57 lies in the consequences of prolonged contact of the supporting element 2b of the medical device 1b with the skin of the patient.

In fact, if clinical conditions (e.g., prolonged loss, difficulties in drainage, problems due to accidental traumas, high density of the fluids to be drained...) occur, the physician is forced to keep the medical device 1b in place for a prolonged period of time, even for several days.

The application of the extension elements 57 makes it possible to position the supporting element 2b of the medical device 1b in a position that is spaced from the exit point of the catheter, so that the patient is allowed to move freely and to assume any comfortable position.

The absence of contact between the supporting element 2b and the skin of the patient prevents the problems of pain and hematoma leading to necrosis, reducing complications and the infective risk.

The placement of the supporting element 2b in an accessible position allows medical and nursing staff to perform all necessary operations quickly and independently of the cooperative capacity of the patient.

In particular, the replacement of the collection system, the execution of diagnostic aspirations with the syringe, the execution of lavages, etcetera... are the operations most frequently required for correct management of the drainage.

The presence of said eyelet elements 59 both on the supporting element 2b and on the elements 55 and 56 allows to fix both parts in different positions if the extension line is used. By doing so, the supporting element 2b can be arranged where it does not cause inconvenience to the patient.

Moreover, besides the described thoracentesis and thoracic drainage procedures, the medical device 1a and 1b is suitable also for pericardiocentesis procedures, where the sharp-tipped needle 13 is inserted in the thorax and in the pericardiac membrane where the heart is contained.

All this is done to remove blood accumulations and avoid cardiac tamponade, i.e., cardiac arrest caused by the pressure of the accumulated fluids.

The structure of the medical devices 1a and 1b is such that they can be used effectively in paracentesis procedures, where the liquids to be drained are located in the abdominal space below the diaphragm.

In practice it has been found that the medical device for applying catheters, particularly for thoracentesis procedures, according to the present invention, fully achieves the intended aim and objects, because it makes it possible to perform thoracentesis procedures without causing damage to the soft tissues of the patient during the application of the catheter, giving constant feedback on the compactness of the tissue that the sharp-tipped needle is penetrating and most of all allowing the repositioning of the catheter inside the pleural space without replacing it and without detaching any syringes or collection bags from the medical device itself.

Another advantage of the medical device according to the present invention consists in that it is easy to use and structurally simple so as to have low manufacturing costs.

Another advantage of the medical device according to the present invention consists in that it is possible to perform aspiration of the fluids to be drained, by means of a syringe, without having to switch the selector valve at each stroke of the piston of the syringe, greatly simplifying this operation.

A further advantage of the medical device according to the present invention consists in the absence of a one-way valve in the rear grip of the sharp-tipped needle, despite maintaining the possibility to perform an exploratory aspiration in order to determine the correct positioning of the needle.

Said valve, in fact, is integrated in the body of the device. Therefore the device is more compact, simpler and easier to handle, entirely to the advantage of the physician who uses it.

The medical device for applying catheters, particularly for thoracentesis procedures, according to the present invention is susceptible of numerous modifications and variations, all of which are within the scope of the appended claims.

For example, the slider, which is accommodated in the container body, contrary to what has been stated so far, can be movable from a non-visible position to a visible one, respectively, in which the round tip protrudes at least partially from the sharp-tipped needle (non-visible position of the slider) and in which the round tip is completely retracted into the sharp-tipped needle (visible position of the slider).

All the details may further be replaced with other technically equivalent elements.

In practice, the materials used, as long as they are compatible with the specific use, as well as the contingent shapes and dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A medical device (1a, 1b) for applying catheters, particularly for thoracentesis procedures, comprising a hollow supporting element (2a, 2b) which can be gripped ergonomically and accommodates a selector (3a, 3b) which is operated manually and is adapted to connect at least three openings (4a, 5a, 6a, 4b, 5b, 6b) defined in said supporting element (2a, 2b), the first one of said openings (4a, 4b) being functionally associated with a cannula (7) that is adapted to be inserted in the body of a patient for the drainage of biological liquid (100) or for the injection of drugs, the second opening (5a, 5b) and the third opening (6a, 6b) being able to be functionally associated respectively with two closure elements (8, 9) or with at least one of a syringe (10) and a collection bag (11), a sharp-tipped needle (13) being slidingly insertable in said cannula (7) and having such a length as to pass through at least all of said cannula (7) and protrude from said cannula (7) to pierce the biological tissue (101) that lies in the vicinity of said biological liquid (100), said device comprising a fourth opening (14a, 14b) defined in said supporting element (2a, 2b) on the side opposite said first opening (4a, 4b) and dedicated only to the operations of extraction and insertion of said sharp-tipped needle (13) respectively from and into said cannula (7) without involving the second and third openings (5a, 5b; 6a, 6b), said device further comprising a first valve (27) of the one-way type, which is normally closed, is accommodated in said supporting element (2a, 2b), is interposed between said selector (3a, 3b) and said fourth opening (14a, 14b), and is provided with a membrane-type sealing gasket (28) that can be engaged by abutment against said container body (18a, 18b), said first valve (27) having a central slit which is elastically deformable for the passage of said sharp-tipped needle (13) in both directions, and said device comprising a second valve (30a, 30b) of the one-way type, which is accommodated in said supporting element (2a, 2b) and is interposed between said selector (3a, 3b) and said second opening (5a, 5b).

2. The medical device (1a, 1b) according to claim 1, **characterized in that** it comprises a round-tipped element (15), which is inserted slidingly in said sharp-tipped needle (13) so that its round tip (16) protrudes from said sharp-tipped needle (13) on the side opposite said supporting element (2a, 2b), said round tip (16) being retractable into said sharp-tipped needle (13) in opposition to the action of elastic means (17) associated with said round-tipped element (15).

3. The medical device (1a, 1b) according to claim 2, **characterized in that** said elastic means (17) comprise a helical spring, which is accommodated in a container body (18a, 18b) that is jointly connected to said sharp-tipped needle (13) and is interposed between one end of said round-tipped element (15) and an internal abutment surface (19a, 19b) of said container body (18a, 18b), said container body (18a, 18b) being engageable with said supporting element (2a, 2b) at said fourth opening (14).

4. The medical device (1a, 1b) according to one or more of the preceding claims, **characterized in that** it comprises means (20) for visualizing the retraction of said round tip (16) into said sharp-tipped needle (13).

5. The medical device (1a, 1b) according to claim 4, **characterized in that** said visualization means (20) comprise a slider (21a, 21b), which is associated with said round-tipped element (15) on the opposite side with respect to said round tip (16), is accommodated in said container body (18a, 18b) and can move from a visible position, in which said round tip (16) protrudes at least partially from said sharp-tipped needle (13), to a non-visible position, in which said round tip (16) is completely retracted into said sharp-tipped needle (13), said container body (18a, 18b) being at least partially transparent for viewing said slider (21a, 21b) in said visible position.

6. The medical device (1a, 1b) according to one or more of the preceding claims, **characterized in that** it comprises manually actuated control means for said selector (3 a, 3b), comprising a control lever (22a, 22b), which can move from a first functional position to a second functional position, said four openings (4a, 5a, 6a, 14a, 4b, 5b, 6b, 14b) being mutually connected when said control lever (22a, 22b) is arranged in said first functional position and said first opening (4a, 4b), said second opening (5a, 5b) and said third opening (6a, 6b) being mutually connected when said control lever (22a, 22b) is arranged in said second functional position.

7. The medical device (1b) according to claim 6, **characterized in that** said manually actuated control means comprise a flap (50) which is rigidly coupled to said control lever (22b) and protrudes along a direction that is substantially parallel to the axis of rotation of said selector (3b) inserted in said supporting element (2b).

8. The medical device (1a, 1b) according to claim 1, **characterized in that** said first valve (27) and said membrane-type sealing gasket (28) are arranged with respect to each other at a distance that is larger than the sum of the length of the sharp part (29) of said sharp-tipped needle (13) and the stroke of said round tip (16) of said round-tipped element (15) to prevent unwanted leaks of said biological liquid (100) to be drained during the operations for inserting and extracting said sharp-tipped needle (13) respectively in and from said cannula (7).

9. The medical device (1a, 1b) according to one or more of the preceding claims, **characterized in that** it comprises at least one groove (12) that is formed on the internal surface of said cannula (7) and runs longitudinally with respect to said cannula (7) for the connection of said first opening (4a, 4b) to said biological liquid (100) to be drained.

10. The medical device (1b) according to one or more of the preceding claims, **characterized in that** it comprises a spindle (51) having a small rod (52), which can be slidingly inserted in said cannula (7) as a substitution for said sharp-tipped needle (13) and has such a length as to pass through at least all of said cannula (7) and protrude from said cannula (7) for repositioning of said cannula (7) in the patient.

11. The medical device (1b) according to one or more of the preceding claims, **characterized in that** it comprises at least one connecting element (54) provided by a male element (55) and a female element (56), which can be mutually associated and fixed, respectively, to said supporting element (2b) at said first opening (4b) and to said cannula (7).

12. The medical device (1b) according to one or more of the preceding claims, **characterized in that** it comprises at least one extension element (57) provided by a small tube (58) provided, at its ends, with said male element (55) and said female element (56) and can be interposed between said male element (55) of said supporting element (2b) and said female element (56) of said cannula (7).

13. The medical device (1b) according to one or more of the preceding claims, **characterized in that** it comprises eyelet elements (59), which are formed in said supporting element (2b) and in said at least one connecting element (54).

## Patentansprüche

1. Medizinische Vorrichtung (1a, 1b) zur Anwendung von Kathetern, im Besonderen für Pleurapunktionsverfahren, umfassend ein hohles Stützelement (2a, 2b), das ergonomisch ergriffen werden kann und einen Selektor (3a, 3b) aufnimmt, der manuell betätigt wird und geeignet ist, um mindestens drei Öffnungen (4a, 5a, 6a, 4b, 5b, 6b) zu verbinden, die in dem genannten Stützelement (2a, 2b) definiert sind, wobei die erste der genannten Öffnungen (4a, 4b) funktionell mit einer Kanüle (7) verbunden ist, die geeignet ist, um in den Körper eines Patienten für die Drainage einer biologischen Flüssigkeit (100) oder zum Injizieren von Arzneimitteln eingeführt zu werden, wobei die zweite Öffnung (5a, 5b) und die dritte Öffnung (6a, 6b) geeignet sind, um jeweils funktionell mit zwei Verschlusselementen (8, 9) oder mit mindestens einem von einer Spritze (10) und einem Auffangbeutel (11) assoziiert zu werden, wobei eine spitze Nadel (13) in die genannte Kanüle (7) gleitend einführbar ist und eine solche Länge aufweist, um durch mindestens die gesamte genannte Kanüle (7) durchzutreten und aus der genannten Kanüle (7) hervorzutreten, um das biologische Gewebe zu durchstechen (101), das in der Nähe der genannten biologischen Flüssigkeit (100) liegt, welche Vorrichtung eine vierte Öffnung (14a, 14b) umfasst, die in dem genannten Stützelement (2a, 2b) auf der der ersten Öffnung (4a, 4b) gegenüberliegenden Seite definiert ist und nur für die Schritte des Zurückziehens beziehungsweise des Einführens der genannten scharfen Nadel (13) aus der und in die genannte Kanüle (7) vorgesehen ist, ohne die zweiten und dritten Öffnungen (5a, 5b; 6a, 6b) einzubeziehen, welche Vorrichtung ferner ein erstes Ventil (27) vom Rückschlagtyp aufweist, das üblicherweise geschlossen ist, in dem genannten Stützelement (2a, 2b) aufgenommen ist, zwischen dem genannten Selektor (3a, 3b) und der genannten vierten Öffnung (14a, 14b) angeordnet ist und mit einer Membran-Dichtung (28) versehen ist, die durch Anschlag gegen den genannten Behälterkörper (18a, 18b) gekuppelt werden kann, wobei das genannte erste Ventil (27) einen zentralen Spalt aufweist, der für den Durchtritt der genannten spitzen Nadel (13) in beide Richtungen elastisch verformbar ist, und welche Vorrichtung ein zweites Ventil (30a, 30b) vom Rückschlagtyp umfasst, welches in dem genannten Stützelement (2a, 2b) aufgenommen ist und zwischen dem genannten Selektor (3a, 3b) und der genannten zweiten Öffnung (5a, 5b) angeordnet ist.

2. Medizinische Vorrichtung (1a, 1b) nach Anspruch 1, **dadurch gekennzeichnet, dass** sie ein Element mit runder Spitze (15) umfasst, das gleitend in die genannte spitze Nadel (13) eingeführt wird, so dass seine runde Spitze (16) aus der genannten spitzen Nadel (13) auf der dem Stützelement (2a, 2b) gegenüberliegenden Seite hervortritt, wobei die runde Spitze (16) in die spitze Nadel (13) gegen die Wirkung des mit dem Element mit runder Spitze (15) assoziierten elastischen Mittels (17) zurückziehbar ist.

3. Medizinische Vorrichtung (1a, 1b) nach Anspruch 2, **dadurch gekennzeichnet, dass** das genannte elastische Mittel (17) eine Schraubenfeder umfassen, die in einem Behälterkörper (18a, 18b) untergebracht ist, der mit der genannten spitzen Nadel (13) zusammenwirkend verbunden ist und zwischen einem Ende des genannten Elements mit runder Spitze (15) und einer inneren Anschlagfläche (19a, 19b) des genannten Behälterkörpers (18a, 18b) angeordnet ist, welcher genannte Behälterkörper (18a, 18b) mit dem genannten Stützelement (2a, 2b) an der genannten vierten Öffnung (14) kuppelbar ist.

4. Medizinische Vorrichtung (1a, 1b) nach einem oder mehreren der vorhergehenden Ansprüche, **dadurch gekennzeichnet, dass** sie ein Mittel (20) zum Sichtbarmachen des Zurückziehens der genannten runden Spitze (16) in die scharfe Nadel (13) aufweist.

5. Medizinische Vorrichtung (1a, 1b) nach Anspruch 4, **dadurch gekennzeichnet, dass** das genannte Mittel (20) zum Sichtbarmachen einen Schieber (21a, 21b) umfasst, der mit dem Element mit runder Spitze (15) auf der in Bezug auf die genannte runde Spitze (16) gegenüberliegenden Seite assoziiert ist, in dem genannten Behälterkörper (18a, 18b) aufgenommen ist und sich von einer sichtbaren Stellung, in der die runde Spitze (16) mindestens teilweise aus der genannten spitzen Nadel (13) hertritt, in eine nicht sichtbare Stellung, in der die genannte runde Spitze (16) vollständig in die genannte spitze Nadel (13) zurückgezogen ist, bewegen kann, welcher Behälterkörper (18a, 18b) mindestens teilweise transparent ist, um den Schieber (21a, 21b) in der genannten sichtbaren Stellung zu sehen.

6. Medizinische Vorrichtung (1a, 1b) nach einem oder mehreren der vorhergehenden Ansprüche, **dadurch gekennzeichnet, dass** sie manuell betätigte Steuermittel für den genannten Selektor (3a, 3b) aufweist, umfassend einen Steuerhebel (22a, 22b), der sich von einer ersten Funktionsstellung zu einer zweiten Funktionsstellung bewegen kann, wobei die vier Öffnungen (4a, 5a, 6a, 14a, 4b, 5b, 6b, 14b) miteinander verbunden sind, wenn der genannte Steuerhebel (22a, 22b) in der genannten ersten Funktionsstellung angeordnet ist, und wobei die genannte erste Öffnung (4a, 4b), die genannte zweite Öffnung (5a, 5b) und die genannte dritte Öffnung (6a, 6b) miteinander verbunden sind, wenn der genannte Steuerhebel (22a, 22b) in der genannten zweiten Funktionsstellung angeordnet ist.

7. Medizinische Vorrichtung (1b) nach Anspruch 6, **dadurch gekennzeichnet, dass** das genannte manuell betätigte Steuermittel eine Klappe (50) umfasst, die starr an den genannten Steuerhebel (22b) gekuppelt ist und entlang einer Richtung vorsteht, die im wesentlichen parallel zur Drehachse des in das genannte Stützelement (2b) eingesetzten Selektors (3b) ist.

8. Medizinische Vorrichtung (1a, 1b) nach Anspruch 1, **dadurch gekennzeichnet, dass** das genannte erste Ventil (27) und die genannte Membran-Dichtung (28) zueinander in einem Abstand angeordnet sind, der größer ist als die Summe aus der Länge des spitzen Teils (29) der genannten spitzen Nadel (13) und dem Hub der genannten runden Spitze (16) des genannten Elements mit runder Spitze (15), um zu verhindern, dass unerwünschte Abflüsse der genannten biologischen Flüssigkeit (100) während der Operationen zum Einsetzen und Herausziehen der genannten spitzen Nadel (13) jeweils in beziehungsweise aus der genannten Kanüle (7) erfolgen.

9. Medizinische Vorrichtung (1a, 1b) nach einem oder mehreren der vorhergehenden Ansprüche, **dadurch gekennzeichnet, dass** sie mindestens eine auf der Innenfläche der genannten Kanüle (7) ausgebildete Nut (12) aufweist, die in Längsrichtung zu der genannten Kanüle (7) verläuft zur Verbindung der genannten ersten Öffnung (4a, 4b) mit der genannten abzuleitenden biologischen Flüssigkeit (100).

10. Medizinische Vorrichtung (1b) nach einem oder mehreren der vorhergehenden Ansprüche, **dadurch gekennzeichnet, dass** sie eine Spindel (51) mit einer kleinen Stange (52) umfasst, die gleitend in die genannte Kanüle (7) als Ersatz für die genannte spitze Nadel (13) eingeführt werden kann und eine solche Länge besitzt, dass sie durch mindestens die gesamte Kanüle (7) durchtritt und aus der genannten Kanüle (7) hervortritt zur Neupositionierung der genannten Kanüle (7) im Patienten.

11. Medizinische Vorrichtung (1b) nach einem oder mehreren der vorhergehenden Ansprüche, **dadurch gekennzeichnet, dass** sie mindestens ein Verbindungselement (54) umfasst, das von einem männlichen Element (55) und einem weiblichen Element (56) bereitgestellt wird, die miteinander verbunden beziehungsweise an dem genannten Stützelement (2b) an der genannten ersten Öffnung (4b) und an der genannten Kanüle (7) befestigt sind.

12. Medizinische Vorrichtung (1b) nach einem oder mehreren der vorhergehenden Ansprüche, **dadurch gekennzeichnet, dass** sie mindestens ein Verlängerungselement (57) aufweist, das von einem schmalen Schlauch (58) bereitgestellt wird, der an seinen Enden mit dem genannten männlichen Element (55) und dem genannten weiblichen Element (56) ausgestattet ist und zwischen dem genannten männlichen Element (55) des genannten Stützelements und dem genannten weiblichen Element (56) der genannten Kanüle (7) angeordnet sein kann.

13. Medizinische Vorrichtung (1b) nach einem oder mehreren der vorhergehenden Ansprüche, **dadurch gekennzeichnet, dass** sie Ösenelemente (59) aufweist, die in dem genannten Stützelement (2b) und in dem genannten mindestens einen Verbindungselement (54) ausgebildet sind.

## Revendications

1. Dispositif médical (1a, 1b) destiné à l'application de cathéters, en particulier pour des procédures de thoracentèse, comprenant un élément de support (2a, 2b) creux qui peut être saisi et reçoit un sélecteur (3a, 3b) qui est actionné manuellement et est adapté pour relier au moins trois ouvertures (4a, 5a, 6a, 4b, 5b, 6b) définies dans ledit élément de support (2a, 2b), la première desdites ouvertures (4a, 4b) étant associée fonctionnellement à une canule (7) qui est adaptée pour être insérée dans le corps d'un patient, en vue du drainage de liquide biologique (100) ou en vue de l'injection de médicaments, la deuxième ouverture (5a, 5b) et la troisième ouverture (6a, 6b) étant aptes à être fonctionnellement associées respectivement à deux éléments de fermeture (8, 9) ou à au moins un élément parmi une seringue (10) et une poche de collecte (11), une aiguille (13) à bout pointu pouvant être insérée par glissement dans ladite canule (7) et ayant une longueur telle qu'elle peut passer dans au moins la totalité de ladite canule (7) et dépasser de ladite canule (7) pour percer le tissu biologique (101) qui se trouve dans le voisinage dudit liquide biologique (100), ledit dispositif comprenant une quatrième ouverture (14a, 14b) définie dans ledit élément de support (2a, 2b), sur le côté opposé à ladite première ouverture (4a, 4b), et réservée uniquement aux opérations d'extraction et d'insertion de ladite aiguille (13) à bout pointu, respectivement hors et dans ladite canule (7), sans faire intervenir les deuxième et troisième ouvertures (5a, 5b ; 6a, 6b), ledit dispositif comprenant en outre une première valve (27) de type anti-reflux qui est normalement fermée, est reçue dans ledit élément de support (2a, 2b), est disposée entre ledit sélecteur (3a, 3b) et ladite quatrième ouverture (14a, 14b) et est pourvue d'un joint d'étanchéité (28) de type membrane qui peut être mis en contact par venue en butée contre ledit corps de récipient (18a, 18b), ladite première valve (27) présentant une fente centrale qui peut être déformée élastiquement pour le passage de ladite aiguille (13) à bout pointu, dans les deux directions, et ledit dispositif comprenant une deuxième valve (30a, 30b) de type anti-reflux qui est reçue dans ledit élément de support (2a, 2b) et est disposée entre ledit sélecteur (3a, 3b) et ladite deuxième ouverture (5a, 5b).

2. Dispositif médical (1a, 1b) selon la revendication 1, **caractérisé en ce qu'**il comprend un élément (15) à bout arrondi qui est inséré par glissement dans ladite aiguille (13) à bout pointu, de sorte que son extrémité arrondie (16) fait saillie à partir de ladite aiguille (13) à bout pointu, sur le côté opposé audit élément de support (2a, 2b), ledit bout arrondi (16) pouvant être rentré dans ladite aiguille (13) à bout pointu, en opposition à l'action de moyens élastiques (17) associés audit élément (15) à bout arrondi.

3. Dispositif médical (1a, 1b) selon la revendication 2, **caractérisé en ce que** lesdits moyens élastiques (17) se composent d'un ressort hélicoïdal qui est disposé dans un corps de récipient (18a, 18b) qui est relié solidairement à ladite aiguille (13) à bout pointu et est placé entre une extrémité dudit élément (15) à bout arrondi et une surface de butée (19a, 19b) interne dudit corps de récipient (18a, 18b), ledit corps de récipient (18a, 18b) pouvant être amené en prise avec ledit élément de support (2a, 2b) à l'endroit de la quatrième ouverture (14).

4. Dispositif médical (1a, 1b) selon une ou plusieurs des revendications précédentes, **caractérisé en ce qu'**il comprend des moyens (20) destinés à la visualisation de la rentrée dudit bout arrondi (16) dans ladite aiguille (13) à bout pointu.

5. Dispositif médical (1a, 1b) selon la revendication 4, **caractérisé en ce que** lesdits moyens de visualisation (20) comprennent un coulisseau (21a, 21b) qui est associé audit élément (15) à bout arrondi, sur le côté opposé par rapport audit bout arrondi (16), est logé dans ledit corps de récipient (18a, 18b) et peut se déplacer à partir d'une position visible, dans laquelle ledit bout arrondi (16) dépasse au moins en partie de ladite aiguille (13) à bout pointu, jusqu'à une position non visible, dans laquelle ledit bout arrondi (16) est complètement rentré dans ladite aiguille (13) à bout pointu, ledit corps de récipient (18a, 18b) étant au moins en partie transparent afin de visualiser ledit coulisseau (21a, 21b) dans ladite position visible.

6. Dispositif médical (1a, 1b) selon une ou plusieurs des revendications précédentes, **caractérisé en ce qu'**il comprend des moyens de commande à actionnement manuel pour ledit sélecteur (3a, 3b), comprenant un levier de commande (22a, 22b) qui peut se déplacer d'une première position fonctionnelle à une deuxième position fonctionnelle, lesdites quatre ouvertures (4a, 5a, 6a, 14a, 4b, 5b, 6b, 14b) étant reliées entre elles lorsque ledit levier de commande (22a, 22b) est placé dans ladite première position fonctionnelle, et ladite première ouverture (4a, 4b), ladite deuxième ouverture (5a, 5b) et ladite troisième ouverture (6a, 6b) étant reliées entre elles lorsque ledit levier de commande (22a, 22b) est placé dans ladite deuxième position fonctionnelle.

7. Dispositif médical (1b) selon la revendication 6, **caractérisé en ce que** lesdits moyens de commande à actionnement manuel comprennent un volet (50) qui est accouplé de façon rigide audit levier de commande (22b) et fait saillie suivant une direction qui est sensiblement parallèle à l'axe de rotation dudit sélecteur (3b) inséré dans ledit élément de support (2b).

8. Dispositif médical (1a, 1b) selon la revendication 1, **caractérisé en ce que** ladite première valve (27) et ledit joint d'étanchéité (28) de type membrane sont disposés l'un par rapport à l'autre à une distance qui est plus grande que la somme de la longueur de la partie pointue (29) de ladite aiguille (13) à bout pointu et de la course dudit bout arrondi (16) dudit élément (15) à bout arrondi, pour empêcher des fuites indésirables dudit liquide biologique (100) devant être drainé, pendant les opérations d'insertion et d'extraction de ladite aiguille (13) à bout pointu, respectivement pour entrer dans ladite canule (7) et en sortir.

9. Dispositif médical (1a, 1b) selon une ou plusieurs des revendications précédentes, **caractérisé en ce qu'**il comporte au moins une rainure (12) qui est formée sur la surface interne de ladite canule (7) et s'étend dans le sens longitudinal par rapport à ladite canule (7), en vue du raccordement de ladite première ouverture (4a, 4b) audit liquide biologique (100) devant être drainé.

10. Dispositif médical (1b) selon une ou plusieurs des revendications précédentes, **caractérisé en ce qu'**il comporte une broche (51) ayant une petite tige (52) qui peut être insérée par glissement dans ladite canule (7), en remplacement de ladite aiguille (13) à bout pointu, et présente une longueur telle qu'elle peut passer dans au moins la totalité de ladite canule (7) et dépasser de ladite canule (7), en vue du repositionnement de ladite canule (7) dans le patient.

11. Dispositif médical (1b) selon une ou plusieurs des revendications précédentes, **caractérisé en ce qu'**il comporte au moins un élément de raccordement (54) constitué d'un élément mâle (55) et d'un élément femelle (56) qui peuvent être associés l'un à l'autre et fixés respectivement audit élément de support (2b), à l'endroit de ladite première ouverture (4b), et à ladite canule (7).

12. Dispositif médical (1b) selon une ou plusieurs des revendications précédentes, **caractérisé en ce qu'**il comporte au moins un élément de prolongement (57), constitué d'un petit tube (58) muni, à ses extrémités, dudit élément mâle (55) et dudit élément femelle (56), et pouvant être disposé entre ledit élément mâle (55) dudit élément de support (2b) et ledit élément femelle (56) de ladite canule (7).

13. Dispositif médical (1b) selon une ou plusieurs des revendications précédentes, **caractérisé en ce qu'**il comporte des éléments formant oeillets (59) qui sont réalisés dans ledit élément de support (2b) et dans ledit élément de raccordement (54), au nombre d'au moins un.
